# EUROPEAN PATENT SPECIFICATION

(11) **EP 2 181 690 B1**
(45) Date of publication and mention of the grant of the patent: **09.11.2011**
(21) Application number: 09161506.2
(22) Date of filing: 29.05.2009
(51) Int. Cl.: A61K 8/06, A61K 8/31, A61K 8/39, A61K 8/58, A61K 8/92, A61Q 13/00

(54) **Perfume composition with reduced alcohol content**
Parfümzusammensetzung mit reduziertem Alkoholgehalt
Composition de parfum avec contenu en alcool réduit

(30) Priority: 29.05.2008 EP 08157158
(43) Date of publication of application: 05.05.2010
(73) Proprietor: Coty Inc., New York, NY 10116 (US)
(72) Inventor: Bleuez, Loïc, 06700, Saint Laurent du Var (FR); Porcu, Maryse, 06240, Beausoleil (FR)
(74) Representative: Gulde Hengelhaupt Ziebig & Schneider

(56) References cited:
- EP-A- 0 571 677
- EP-A- 0 701 813
- WO-A-00/33804
- WO-A-2005/051339
- WO-A-2006/012767
- US-A- 4 781 914
- US-A1- 2005 032 916

## Description

The invention relates to perfume compositions with high perfume oil content and reduced or no alcohol content of C₂-C₅ monovalent alcohols, especially ethanol. The compositions comprise a stable transparent or transluscent emulsion with an oil and water phase.

### Background of the Invention

Lower alcohols, primarily ethanol, is the most interesting solvent of fragrance oil in perfumes. Ethanol evaporates quickly into the atmosphere, cools the skin and conveys a very desirable refreshing feeling on the human skin.

Regulations for environmental protection have recently become more stringent, also regarding volatile organic compounds. For this reason, one of the aims of the cosmetic industry is to reduce the high content of ethanol in perfumes or to replace it completely.

EP 0687460 B1 refers to low-alcohol perfume compositions comprising a lower glycol having up to six carbon atoms and/or a lower alcohol having four to six carbon atoms and at least four carbon atoms in its principal chain, and further comprising 40 - 75 per cent by weight of ethyl alcohol.

US 5,736,505 discloses a non-alcoholic fragrance comprising a hydrophobic perfume base, water, a non-ionic surfactant and glycereth-7 triacetate.

US 6,774,101 refers to an alcohol-free translucent perfuming composition with a surfactant of HLB > 10 being polyethylenglycol ethers and heavy paraffins. A preparation temperature of 75 - 95 °C is disadvantageous for that composition.

US 2007/0178144 refers to cosmetic O/W emulsions comprising at least one non-ionic primary emulsifier, at least one secondary emulsifier, one or more cosurfactants and usual cosmetic oils with the proviso that the water phase content of the emulsion is at least 70 wt-%.

WO 2000/33804 refers to perfume compositions which are free of volatile organic solvents. Transparency of these compositions is achieved by levelling the refractive indices of the oil phase and the water phase until the difference is smaller than 0.003.

The object of the present invention is to provide translucent or transparent perfume compositions with reduced or no lower alcohol content but high perfume oil contents in a long-lasting stable preparation and storable in a broad temperature range.

A further object is to provide a complete olfactory restitution of fragrance oil connected with a long-time olfactory stability.

A further object is to provide perfume compositions with conveying a fresh feeling and without stickiness on the skin.

### Summary of the Invention

The perfume composition of the invention with reduced alcohol content comprises a transparent or translucent emulsion with an oil phase and a water phase wherein
(a) the oil phase comprises 25 to 95 wt-% of a perfume oil or perfume oil mixture, and 5 to 75 wt-% of a solvent, the percentages relating to the total weight of the oil phase, wherein the solvent has a log P value of >5 and is selected from Isododecane, Isohexadecane, Isoeicosane, isoparaffine fluids, C₁₃-C₃₀ Alkanes, Hydrogenated Didecene, Hydrogenated Didodecene, Hydrogenated Polydecene, Hydrogenated Polydodecene, Hydrogenated Tridodecene, Hydrogenated Polyisobutene, mineral oils, and a mixture of two or more thereof, or the solvent is a linear silicone, a cyclic silicone or a mixture thereof,
(b) the water phase comprises an emulsifier and a buffer system, wherein the emulsifier is a mixture of a first non-ionic emulsifier, an anionic emulsifier and optionally a second non-ionic emulsifier
(c) the amount of the perfume oil or perfume oil mixture is in the range of 1 to 35 wt-%, related to the total weight of the perfume composition;
(d) the perfume composition comprises aromatic substances having a logP value from 0.5 to 2
(e) the amount of mono valent C₂-C₅ alcohols in the perfume composition is in the range of 0 to 5 wt-% , related to the total weight of the perfume composition; and
(f) the water phase and/or oil phase comprise further cosmetically acceptable ingredients.

The perfume compositions of the invention are an oil-in-water (O/W) emulsions. The smaller the particle diameter of the dispersed droplets the higher is the transparency of the emulsion. Translucency of the emulsion is achieved by a mean particle diameter of the dispersed phase of less than 100 nm. According to the invention stable transparent perfume compositions are achieved using the buffer in the aqueous phase and the special combination of the emulsifiers.

The term "perfume composition" refers to a product of usual perfumes but also to other fragrances such as Eau de Cologne, Eau de Toilette, pre shaves, after shaves, face waters, tonics etc..

"Perfume oils" refers to known substances for modifying the smell/odour of a product or providing a smell/odour to a person. Mixtures of natural essential oils or synthetic fragrances are possible.

The oil phase of the composition comprises perfume oils, essential oils or a mixture thereof. Essential oils which are smelling substances derived by physical processes, preferred distillations, from plants or spices are also subsumed under the term "perfume oils".

Examples of perfume oils are extracts of flowers (e.g. lily, lavender, rose, jasmine, neroli or ylang-ylang), stems and leaves (e.g. geranium, patchouli or petitgrain), fruits (e.g. anis, coriander, cumin or juniper), fruit skins (e.g. bergamot, citrus or orange), roots (e.g. macis, angelica, cardamom, iris or calmus) wood (e.g. pine, sandalwood, guaiac, cedar or rose), herbs and grasses (e.g. tarragon, lemon grass, salvia or thyme), needles and branches (e.g. pine or fir), resins and/or balms (e.g. galbanum, elemi, benzol, myrrh, olibanum or opoponax). Further, animal raw materials such as zibet and/or castoreum can be used as perfume oils according to the invention. Typical synthetic fragrances are for instance products from the ester type, ether type, aldehyde type, ketone type, alcohol type and/or hydrocarbon type.

Useful perfume oils include Galaxolide (hexamethylhexahydrocyclopentabenzopyran, 50 % of Isopropyl Myristate), Ethylene Brassylate (1,4-dioxacycloheptadecan-5,17-dione), Habanolide (CAS-No.: 423773-57-3), Globanone (cyclohexadec-8-en-1-one), Musk Ketone (1-(4-tert-butyl-2,6-dimethyl-3,5-dinitrophenyl)-ethanone), Musk Xylol (1-tert-butyl-3,5-dimethyl-2,4,6-trinitrobenzene), Trimofix O (2,5,10-trimethyl-2,5,9-cyclododecatrien-1-yl methyl ketone & isomers), Sandalore (5-(2,2,3-trimethyl-3-cyclopentenyl)-3-methylpentan-2-ol), Boisambrene (ethoxymethyl-cyclododecyl ether,), Nerolidol (3,7,11-trimethyl-1,6,10-dodecatrien-3-ol), Cedramber (cedryl methyl ether), Iso E Super (1-(1,2,3,4,5,6,7,8-octahydro-2,3,8,8-tetramethyl-2-naphthalenyl)-ethan-1-one), Irisantheme ((E)-3-methyl-4-(2,6,6-trimethyl-1-cyclohex-2-enyl)but-3-en-2-one), Damascone Alpha ((E)-1-(2,6,6-trimethyl-1-cyclohex-2-enyl)but-2-en-1-one), Orange Bresil (essential oil, for instance available from Eau-Douce, France), Orange EO from Brazil (CAS: 8028-48-6), Lilial (lNCl name: butylphenyl methylpropional), Aldehyde Alpha Hexyl Cinnamic (hexyl cinnamal), Farnesol (3,7,11-trimethyl- 2,6,10-dodecatrien-1-ol), Bacdanol (2-ethyl-4-(2,2,3-trimethyl-3-cyclo-penten-1-yl)-2-buten-1-ol), Ebanol (3-methyl-5-(2,2,3-trimethyl-3-cyclopenten-1-yl)-4-penten-2-ol), Guaiac Wood Acetate (CAS 61789-17-1), Cedrenyl Acetate (CAS-No.: 1405-92-1), Hexyl Salicylate, Verdox (2-tert-butylcyclohexyl acetate), Cis-3 Hexenyl Salicylate, Linalool, (3,7-dimethyl-1,6-octadien-3-ol,) Linalyl Acetate (CAS-No.: 115-95-7) or Benzyl Salicylate, Hedione (methyl dihydrojasmonate), Dihydromyrcenol (2,6- dimethyl-7-octen-2-ol) and mixtures thereof. Other perfume oils are also possible according to Int. Cosmetic Ingredient Dictionary and Handbook, 12th ed. 2008, Vol. 3,p. 3193-3200 (edited by CTFA, Washington DC, USA).

To obtain a fragrance mixture with a combination of perfume oils it is preferred to complete the perfume oils by some aromatic (odorant) substances. Aromatic substances according to the invention are preferably raw materials having a log P value from 0.5 to 2, more preferred from 0,5 to 1,5, most preferred from 0,7 to 1,3. Preferred raw materials are for instance alcohols e.g. benzyl alcohol, cis-3-hexenol, phenyl ethyl alcohol, methylbenzyl alcohol, anisyl alcohol, isoamyl alcohol, 4-hexen-1-ol, phenoxyethanol, phenoxypropanediol, trimethyl-1,3-pentanediol, chlorphenesin, ethylhexyl glycerin, caprylyl glycol, glyceryl caprylate, hexanediol, 1,2-hexane diol, ethyl hexanediol, pentylene glycol, octanediol, hydroxypropyl methacrylate, triethyl citrate or mixtures thereof.

According to the invention the definition of aromatic (odorant) substances also comprises such substances as e.g. ethyl vanilline, vanilline (4-hydroxy-3-methoxybenzaldehyd), heliotropine (1,3-benzodioxole-5-carboxaldehyde), helional (alphamethyl-1,3-benzodioxole-5-propanal), coumarin (1,2-Benzopyrone), ethyl maltol (2-ethyl pyromeconic acid), ethyl acetate, ethyl acetoacetate, 2-methyl pyrazine, linalool oxide (6-methyl-2-(oxiran-2-yl)hept-5-en-2-ol), hydroxycitronellal (7-hydroxy-3,7-dimethyloctanal), ethylphenol, benzyl acetate, allyl amyl glycolate or mixtures thereof.

According to the invention the perfume composition preferably comprises as aromatic substances aromatic alcohols, more preferred benzyl alcohol, phenoxyethanol, phenoxy propanol, phenoxy propanediol, phenyl ethyl alcohol, methylbenzyl alcohol, anisyl alcohol or mixtures thereof, most preferred phenoxyethanol and/or benzyl alcohol with a log P value of 1,08 - 1,1.

The aromatic substances are contained in the perfume oil composition preferably from 0,2 to 7,5 wt-%, more preferred from 0,3 to 6 wt-%, most preferred from 0,5 to 3 wt-% related to the total weight of the composition.

The term "reduced alcohol content" means that the content of monovalent C₂-C₅ alcohols such as for instance ethanol is in the range of 0 - 5 wt-%, preferably 0 - 2 wt-% and most preferred 0 wt-% related to the total weight of the perfume composition.

It is also possible to add to the oil phase such substances as Isononyl lsononanoate, Octyldocenol, linear silicones of the Dimethicone type or Diethyl Hexyl Carbonate, esters of myristic acid, e.g. Isopropyl Myristate, Ethylhexyl Palmitate, PG, DPG, TPG, DPPG, Ethyl Phtalate, Ethyl Hexyl Stearate, Decyl Cocoate, Dioctyl Carbonate, Cetearyl Ethyl Hexanoate, Decyl Oleate, Isocetyl Palmitate, Cetearyl Isononanoate, Myristyl Myristate, Hexyl Laurate, Isopropyl Isononanoate, Isopropyl Palmitate, Isopropyl Laurate, C₁₂-₁₅ Alkyl Benzoate, pentaerythritol esters (e.g. Pentaerythrityl Tetracaprytate/Tetracaprate and/or Dipentaerythrityl Pentaisononanoate). These substances may be contained up to 3 wt-% related to the total weight of the composition.

The solvents in the oil phase preferably have a calculated log P value in the range of 5 to 12, more preferred in the range of 5 to 9, where P stands for the octanol/water partitioning coefficient of a material, i.e. the ratio between its equilibrium concentration in octanol and water, which is a measure of hydrophobicity. High partitioning coefficient values are more conveniently given in the form of their logarithm to the base 10, log P. While log P values can be measured experimentally, and measured log P data is available for many perfumes, log P values are most conveniently calculated. There are several recognised calculation or estimation methods available commercially and/or described in the literature (see for example A. Leo, Chem. Rev. 93(4), 1281-1306, (1993), "Calculating log P oct from structures"). Generally these models correlate highly but may for specific materials produce log P values which differ in absolute terms (by up to 0.5 log units or even more). However, no one model is universally accepted as the most accurate across all compounds. This is particularly true for estimates on materials of high log P (about 5 or greater). In the present specification, calculated log P values are obtained using the estimation software of Syracuse Research Corp., SRC, which is well-known to the scientific community, and accepted as providing high-quality predictions of log P values. References to calculated log P values thus mean values obtained using the a.m. software.

According to the invention most preferred solvents are Isohexadecane (log P 7.79), C₁₅-C₁₉ Alkanes or C₁₃-C₁₆ Isoparaffins (log P 7.63), or mixtures thereof. Other useful solvents are Isoeicosane (log P 10.09), Isododecane (log P 6.16) and/or mineral oil (e.g. Paraffin oil ISO W 05 supplied by AIGLON). The choice of the solvent according to the invention is important because a log P value outside the given ranges would destabilize the perfume emulsion according to the invention.

Preferably the oil phase comprises 35 to 85 wt-%, more preferred 40 to 70 wt-% of the perfume oil or perfume oil mixture and 10 to 60 wt-%, more preferred 20 to 45 wt-% of the solvent, related to the total weight of the oil phase.

The amount of the perfume oil is in the range of 1 - 35 wt-%, preferably 5-25 wt-%, more preferred 7-15 wt-% related to the total weight of the perfume composition.

The amount of the solvent is usually in the range of 0,1 to 20 wt-%, preferably of 0,5 to 15 wt-%, more preferred of 2 to 10 wt-% related to the total weight of the composition.

In a preferred embodiment of the invention the weight of the oil phase is in the range of 2 to 50 wt %, preferably in the range of 5 to 30 wt % and most preferred in the range of 10 to 15 wt % based on the total weight of the composition.

According to the invention the first non-ionic emulsifier is selected from the group consisting of glyceryl partial esters, polyglyceryl partial esters, sorbitan partial esters, sorbitol partial esters, carbohydrate esters, (alkylpoly)glycosides or mixtures thereof. The carbocyclic acids and alcohols used for the preparation of these esters are preferably C₆-C₂₂ carbocyclic acids and C₆-C₂₂ alcohols. According to the invention the first non-ionic emulsifier is preferably a polyglyceryl partial ester.

Preferred polyglyceryl partial esters according to the invention are for example Polyglyceryl-2 Laurate, Polyglyceryl-3 Laurate, Polyglyceryl-4 Laurate, Polyglyceryl-5 Laurate, Polyglyceryl-6 Laurate, Polyglyceryl-10 Laurate, Polyglyceryl-4 Dilaurate, Polyglyceryl-4 Stearate, Polyglyceryl-4 Isostearate, Polyglyceryl-4 Cocoate, Polyglyceryl-4 Oleate and/or Polyglyceryl-4 Caprate. Non-ionic emulsifiers with ethoxylated branches can also be used. In an especially preferred embodiment of the invention Polyglyceryl-4-Laurate is used as the first non-ionic emulsifier.

The first non-ionic emulsifier is present in the perfume composition of the invention from 0,5 to 16 wt-%, preferably from 0,8 to 12 wt-%, more preferred from 0,8 to 8 wt-% related to the total weight of the composition.

Preferred anionic emulsifiers which are used in mixture with the first non-ionic emulsifier are e.g. sulfates, sulfonates, citrates, sulfosuccinates, succinates, tartrates and/or phosphates esterified with C₆-C₂₂ alcohols. Suitable examples according to the invention are dilauryl citrate, disodium lauryl sulfosuccinate, disodium laureth sulfosuccinate, trisodium sulfosuccinate, disodium PEG-5 laurylcitrate sulfosuccinate, sodium laureth sulfate, sodium lauryl sulfate, sodium trideceth sulfate, sodium lauroyl glutamate and/or sodium cocoyl glutamate or mixtures thereof. The anionic emulsifier can be neutralised with alkaline metal salts or alkaline earth metal salts, e.g. Na or K. Anionic emulsifiers with ethoxylated branches can also be used.

The anionic emulsifier of the invention is contained in the composition in a range from 0,1 to 2,5 wt-%, preferably from 0,2 to 1,5 wt-%.

Especially preferred emulsifiers are e.g. a mixture of Polyglyceryl-4 Laurate (e.g. Tego® Care PL4 of Degussa) and Dilauryl Citrate and Disodium Lauryl Sulfosuccinate (e.g. Rewopol® SB F 12 P of Degussa) or a mixture of Polyglyceryl-4 Laurate and Disodium Lauryl Sulfosuccinate or a mixture of Polyglyceryl-3 Laurate (e.g. Hydramol® TGL Ester of Noveon) and Polyglyceryl-4 Laurate and Sorbitan Laurate (e.g. Tego® SML of Degussa) and Disodium Lauryl Sulfosuccinate.

The inventor has also found that it is advantageous to use an anionic surfactant, in particular disodium laureth sulfosuccinate (e.g. Rewopol SB FA 30), alone or in mixture with disodium lauryl sulfosuccinate.

In a further preferred embodiment of the invention a second non-ionic emulsifier can be added to the composition of the invention. Suitable second non-ionic emulsifiers which can be used are for instance polyethylene glycol hydrogenated castor oil with 2 to 200 polyethylene glycol units, preferably PEG-40 hydrogenated castor oil and/or PEG-60 hydrogenated castor oil, polysorbate 20, polysorbate 40, polysorbate 60, oleth-5, oleth-10, oleth-20 or PPG-1-PPG-9 lauryl glycol ether and mixtures thereof. PEG-40 hydrogenated castor oil and/or PEG-60 hydrogenated castor oil are preferred according to the invention. It is advantageous to add a second non-ionic emulsifier to the perfume composition of the invention, because the addition can improve (fine tune) the properties of the emulsion, in particular stability of the emulsion, transparency and/or translucency as well as freeze/thaw stability.

If a second non-ionic emulsifier is present in the perfume composition of the invention, it is present in a range of 0.1 to 7 wt-%, preferably in a range of 0.5 to 4 wt-% and more preferred in a range of 0,5 to 2 wt-% based on the total weight of the composition.

Further emulsifiers, such as amphoteric emulsifiers, e.g. cocamidopropyl betaine, capryl/capramidopropyl betaine, undecylenamidopropyl betaine, disodium cocoamphodiacetate, sodium cocoamphoacetate or disodium lauroamphodiacetate and mixtures thereof may optionally be added to the perfume composition of the invention. If amphoteric emulsifiers are used they are added in the range from 0.1 to 5 wt-% without changing the stability and transparency of the composition.

The range of the total emulsifier is usually from 0.6 to 20 wt-%, preferably from 1 to 12 wt-%, more preferred from 3 to 10 wt-% related to the total weight of the composition.

The buffer system in the water phase plays a very important role for the stability of the whole emulsion composition.

By the buffer system the well-known pH-drop which is described for aromatic substances due to, for example, some ester hydrolysis (linalyle acetate, benzyl acetate or allyl amyl glycolate) or any aldehyde oxidation (aldehyde α-hexyl cinnamic, helional or lilial) is prevented.

The buffer system comprises at least one inorganic or organic component and preferably a mixture of one or several inorganic and/or organic components.

In a preferred embodiment of the invention the buffer system is selected from sodium dihydrogenphosphate/disodium hydrogenphosphate, citric acid/trisodium citrate, disodium citrate/HCl, potassium hydrogen phthalate/HCl, (potassium hydrogen phthalate/HCl)/NaOH, (disodium citrate/HCl)/NaOH, potassium dihydrogen phosphate/disodium hydrogen phosphate, sodium barbital/HCl, borax solution/HCl, ((glycine+NaCl)/HCl)/NaOH, citric acid/disodium hydrogen phosphate, sodium acetate/acetic acid, imidazole/HCl, triethanol - amine + titriplex III/HCl, tris(hydroxymethyl) aminomethane/HCl or sodium - carbonate/sodium hydrogen carbonate or mixtures thereof.

Especially preferred according to the invention are sodium dihydrogenphosphate/- disodium hydrogenphosphate as well as citric acid/trisodium citrate.

In a preferred embodiment of the invention the buffer system is present in a range of 0.01 to 6 wt-%, preferably 0.4 to 3 wt-%, more preferred 0.4 to 1.5 wt-% related to the total weight of the perfume composition.

Until now it has only been possible to formulate chemically stable perfume oils in water emulsions. It was almost impossible to obtain a stable nanoemulsion containing more than 0.1 wt-% of any aqueous buffer components. This refers to a one-month test in an oven at 45 °C, which is a usual stability test. In contrast the O/W-emulsions of the invention show long-term stability. At elevated temperature over two month no alterations in the size distribution of the dispersed droplets were detected and an increased olfactory restitution of the fragrance's typical scent was noted.

In this connection, the term "stable emulsion" means a particle size profile of the droplets of the dispersed phase of the emulsion maintained for at least two months at room temperature. Analyses were made with the Dynamic Light Scattering (DLS) method, in which the coefficient of diffusion of the particles in solution was measured. Sample solutions were diluted by factor 10 with de-ionised water. The measuring took place by dynamic light scattering with a Malvern HPPS 3.1 at 25 °C for a duration of 100 seconds. The coefficient can be converted by the Stokes-Einstein equation to a mean hydrodynamic radius of the emulsion droplets (equal to particles).

The particle size of the droplets of the present invention are in the range of 5 nm to 1 µm; this means the oil droplets in the water phase or the water droplets in the oil phase. Preferred ranges are from 5 to 200 nm, more preferred from 5 to 100 nm, especially from 10 to 50 nm.

The perfume composition of the present invention can be a milky white emulsion, a translucent emulsion or a transparent emulsion. The emulsion is preferably a translucent to transparent emulsion, more preferred a transparent emulsion with a transmission of more than 90 % to 100 %. Transparent emulsions have a particle diameter of 2 to 35 nm, translucent emulsions have a particle diameter of 35 to 95 nm.

The present invention provides a technique for preparing a low-viscosity, low skin-irritating, fine and prolonged-stability emulsion containing a large amount of perfuming compound, as typically used for impregnating fragrance emulsions, sprayable low alcohol or alcohol-free perfuming emulsions or lotions.

Further important improvements are:
(1) Without the presence of lower alcohols or with largely reduced alcohol contents, a significantly better olfactory restitution of the fragrance's typical scent is possible, along with a longer lasting effective stability as compared to e.g. common Eau de Toilettes.
(2) The special selection of the components of the inventive perfume composition does not require high temperatures for the preparation of the emulsions, so that losses or partial losses of temperature-sensitive perfume oils or essential oils are avoided.
(3) Perfume compositions with high loads of fragrance parts in transparent or translucent quality are possible.
(4) A long-term stability of the present invention's emulsions over the very broad temperature range from -20°C to +45°C/55°C was established in the accelerated aging test.
(5) The emulsions of the invention have a very good feeling to the skin and greatly reduce the feeling of stickiness due to the low content of surfactant.
(6) The emulsions are skin-friendly and provide only a very low irritation because of low surfactant concentrations.

The inventive preparation can also contain further cosmetic auxiliary and carrier substances as they are used conventionally in such compositions, for example, water, floral waters, preservatives, colourings, chelating agent such as EDTA and the like, polyols (such as glycerine, polyglycols, PEG-400, sorbitol), UVA and UVB filters, gelling substances, tanning agents, polymers, copolymers, stabilisers and mixtures thereof, with the proviso that only substances for maintaining a translucent or transparent composition be selected.

The composition according to the invention can also advantageously contain antioxidants, quencher molecules and radical scavengers. Such substances include vitamins such as vitamin C and derivatives thereof, for example, ascorbic acetate, ascorbic phosphate, and ascorbic palmitate; vitamin A and derivatives thereof; folic acid and derivatives thereof; vitamin E and derivatives thereof such as Tocopheryl Acetate, Tocopheryl Linoleate, Tocopheryl Phosphate; flavones or flavonoids; amino acids, such as histidine, glycine, tyrosine, tryptophan, and derivatives thereof; carotenoids and carotenes such as α-carotene, β-carotene and mixtures thereof, with the same proviso as above. Well-known antioxidants are for example BHT, BHA, Tinogard® TT, Tinogard® TS, Tinogard® NOA (from Ciba), TBHQ, Propyl Gallate, Vitamin E TPGS (Tocophersolan), Parsol Guard® (from DSM) and various mixtures known under Covi-OX® (from Cognis) or Oxynex® (from MERCK); Quenchers as Tinoguard® Q (from Ciba), Spectrasolv® (from The Hallstar Company) or Oxynex® ST (from Merck).

In a preferred embodiment the perfume composition of the invention is prepared by combining in one phase the perfume oil or perfume oil mixture, the first non-ionic emulsifier, the one anionic emulsifier, the solvent and optional a humectant and water. The other phase comprises water and the buffer. The two phases were premixed separately and then slowly combined under continuous stirring.

According to the invention all components and/or mixtures of components mentioned in separate preferred embodiments can also be used as combinations of components in one embodiment.

The perfume compositions according to the invention can be used in the form of e.g. perfumes, Eau de Toilettes, Eau de Colognes, sun screen gels, after sun products, lotions, body gels, after shaves, pre shaves, Colognes, moisturizing Eau de Toilette, hair waters and/or facial tonics.

The manufacture of such products is carried out in a way known to a person skilled in the art.

Below, the invention will be described in detail by examples. If not specified otherwise, all percentages are weight percentages.

The accompanying drawings show
- Fig. 1: Exemplified graph with long-term stability of the emulsion according to Example 4 with intensity-weighted size distribution against the particle radius (r)
- Fig. 2: Exemplified graph for the stability of the emulsion according to Example 14 with intensity-weighted size distribution against the particle radius (r)

### Example 1 Perfume I I

| **Phase A** | |
|---|---|
| Water | 4.0 |
| Polyglyceryl-4 Laurate | 4.6 |
| Isohexadecane | 3.8 |
| Fragrance mixture with perfume oils (6.5) (inter alia Iso E Super, Lilial) and aromatic alcohols (1.5) (Benzyl Alcohol and 2-Phenoxyethanol) | 8.0 |
| Glycerine | 2.5 |
| Disodium Lauryl Sulfosuccinate | 0.5 |

| **Phase B** | |
|---|---|
| Water | q.s. ad 100 |
| Sodium Dihydrogenphosphate | 0.8 |
| Disodium Hydrogenphsophate | 0.2 |

The ingredients of phase A are mixed at about 50°C with 350-450 rpm to reach a homogenous mixture. Phase B ingredients are mixed separately to dissolve the salts in water. After that phase B is added slowly to phase A over about 15 - 30 minutes with an increasing stirring rate of 350 to 1000 rpm. Homogenisation at 900 - 1000 rpm for 10 - 20 minutes is possible. Perfume I is a translucent emulsion.

### Example 2 Perfume II

| **Phase A** | |
|---|---|
| Water | 4.4 |
| Polyglyceryl-4 Laurate | 4.6 |
| Isohexadecane | 3.8 |
| Fragrance mixture with perfume oils (6.0)¹ and aromatic alcohols (1.5) (Benzyl Alcohol and 2-Phenoxyethanol) | 7.5 |
| Glycerine | 2.5 |
| Disodium Lauryl Sulfosuccinate | 0.5 |

| **Phase B** | |
|---|---|
| Water | q.s. ad 100 |
| Sodium Dihydrogenphosphate | 0.8 |
| Disodium Hydrogenphsophate | 0.2 |

| | |
|---|---|
| ¹ Mixture of Galaxolide®, Iso E Super®, Habanolide®, Hedione®, Orange EO from Brazil, Dihydromyrcenol, Benzyle salicylate, Linalool, Linalyle acetate, Helional®, Alpha Damascone, Ald. Alpha Hexyl cinnamic. | |

The procedure is according to Example 1. Perfume II is a translucent emulsion.

### Example 3 Perfume III

| **Phase A** | |
|---|---|
| Water | 15.0 |
| Polyglyceryl-4 Laurate | 16.0 |
| Isohexadecane | 13.5 |
| Fragrance mixture with perfume oils (24.5) (Perfect Match 5116259) and aromatic alcohols (5.5) (Benzyl Alcohol and 2-Phenoxyethanol) | 30.0 |
| Glycerine | 9.2 |
| Disodium Lauryl Sulfosuccinate | 1.5 |

| **Phase B** | |
|---|---|
| Water | q.s. ad 100 |
| Sodium Dihydrogenphosphate | 0.15 |
| Disodium Hydrogenphsophate | 0.05 |

The procedure is according to Example 1. Perfect Match 5116259 was from International Flavors & Fragrances Inc. (IFF). Perfume III is a transparent emulsion.

### Example 4 Perfume IV

| **Phase A** | |
|---|---|
| Water | 4.0 |
| Polyglyceryl-4 Laurate | 4.5 |
| Isohexadecane | 3.8 |
| Fragrance mixture with perfume oils (6.8) (Perfect Match 5116259) and aromatic alcohols (1.5) (Benzyl Alcohol and 2-Phenoxyethanol) | 8.3 |
| Glycerine | 2.5 |
| Disodium Lauryl Sulfosuccinate | 0.5 |

| **Phase B** | |
|---|---|
| Water | q.s. ad 100 |
| Sodium Dihydrogenphosphate | 0.8 |
| Disodium Hydrogenphsophate | 0.2 |

The procedure is according to Example 1. Perfume IV is a translucent emulsion.

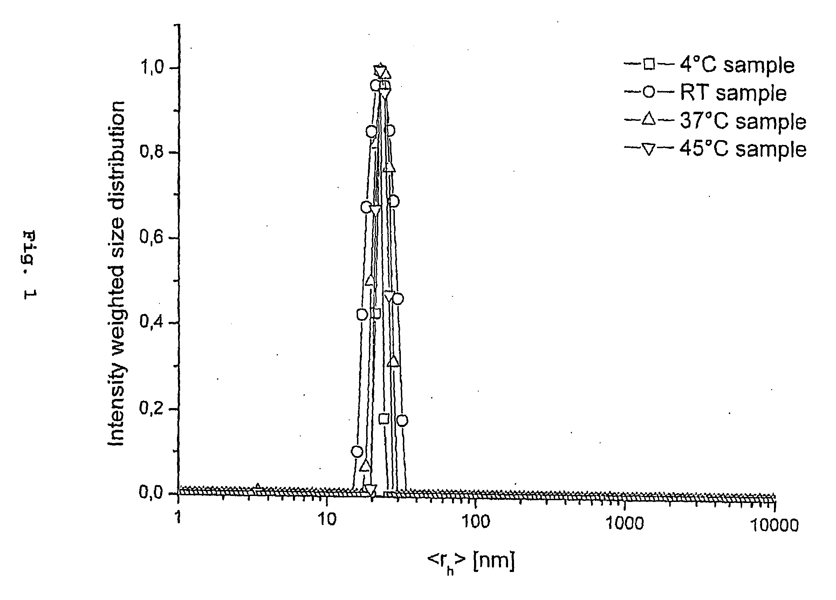

The graph of Fig. 1 shows that samples with different storage temperatures of 4°C, 20°C, 37°C, and 45°C after eight weeks storage time have no alterations in the size distribution. This underlines the unique long-term stability of the emulsion. Additionally, an increased olfactory restitution of the fragrance's typical scent is to be noted.

### Example 5 Perfume V

| **Phase A** | |
|---|---|
| Water | 4.0 |
| Polyglyceryl-4 Laurate | 4.6 |
| C₁₃-C₁₆ Isoparaffins | 4.0 |
| Fragrance mixture with perfume oils (7.0) (Perfect Match 5116259) and aromatic alcohols (1.5) | 8.5 |
| Glycerine | 2.5 |
| Disodium Lauryl Sulfosuccinate | 0.4 |

| **Phase B** | |
|---|---|
| Water | q.s. ad 100 |
| Sodium Dihydrogenphosphate | 0.8 |
| Disodium Hydrogenphsophate | 0.2 |

The procedure is according to Example 1. Perfume V is a translucent emulsion.

### Example 6 Perfume VI

| **Phase A** | |
|---|---|
| Water | 6.0 |
| Polyglyceryl-4 Laurate | 4.5 |
| Isohexadecane | 3.8 |
| Fragrance mixture with perfume oils (6.5) and aromatic alcohols (1.5) | 8.0 |
| Glycerine | 3.0 |
| Disodium Lauryl Sulfosuccinate | 0.4 |

| **Phase B** | |
|---|---|
| Water | q.s. ad 100 |
| Sodium Dihydrogenphosphate | 0.75 |
| Disodium Hydrogenphsophate | 0.25 |
| Ethanol | 5.0 |

The procedure is according to Example 1. Perfume VI is a translucent emulsion.

### Example 7 Perfume VII

| **Phase A** | |
|---|---|
| Water | 4.0 |
| Polyglyceryl-4 Laurate | 4.8 |
| Hydrogenated Polydecene | 3.9 |
| Fragrance mixture with perfume oils (6.5) and aromatic alcohols (1.5) | 8.0 |
| Glycerine | 3.0 |
| Disodium Lauryl Sulfosuccinate | 0.4 |

| **Phase B** | |
|---|---|
| Water | q.s. ad 100 |
| Sodium Dihydrogenphosphate | 0.8 |
| Disodium Hydrogenphsophate | 0.2 |

The procedure is according to Example 1. Perfume VII is a translucent emulsion.

### Example 8 Perfume VIII

| **Phase A** | |
|---|---|
| Water | 1.0 |
| Polyglyceryl-4 Laurate | 0.8 |
| Isohexadecane | 0.6 |
| Fragrance mixture with perfume oils (1.0) and aromatic alcohols (0.3) (Benzyl Alcohol and 2-Phenoxyethanol) | 1.3 |
| Glycerine | 0.5 |
| Disodium Lauryl Sulfosuccinate | 0.1 |

| **Phase B** | |
|---|---|
| Water | q.s. ad 100 |
| Potassium Dihydrogenphosphate | 1.0 |
| Disodium Hydrogenphsophate | 0.3 |

The procedure is according to Example 1. Perfume VIII is a translucent emulsion.

### Example 9 Perfume IX

| **Phase A** | |
|---|---|
| Water | 3.5 |
| Polyglyceryl-4 Laurate | 4.8 |
| Isohexadecane | 4.0 |
| Fragrance mixture with perfume oils (8.0) and aromatic alcohols (2.0) | 10.0 |
| Glycerine | 2.0 |
| Disodium Lauryl Sulfosuccinate | 0.5 |

| **Phase B** | |
|---|---|
| Water | q.s. ad 100 |
| Citric Acid | 0.1 |
| Trisodium Citrate | 0.9 |

The procedure is according to Example 1. Perfume IX is a translucent emulsion.

### Example 10 Perfume X

| **Phase A** | |
|---|---|
| Water | 6.0 |
| Polyglyceryl-4 Laurate | 4.6 |
| Isohexadecane Fragrance mixture with perfume oils (7.0) and | 3.8 |
| aromatic alcohols (1.5) | 8.5 |
| Glycerine | 3.0 |
| Disodium Lauryl Sulfosuccinate | 0.4 |

| **Phase B** | |
|---|---|
| Water | q.s. ad 100 |
| Sodium Dihydrogenphosphate | 0.8 |
| Disodium Hydrogenphosphate | 0.2 |

The procedure is according to Example 1. Perfume X is a translucent emulsion.

### Example 11 Perfume XI

| **Phase A** | |
|---|---|
| Water | 9.0 |
| Polyglyceryl-4 Laurate | 10.0 |
| Isohexadecane | 8.0 |
| Fragrance mixture with perfume oils (15) and aromatic alcohols (3.5) (Benzyl Alcohol and 2-Phenoxyethanol) | 18.5 |
| Glycerine | 5.0 |
| Disodium Lauryl Sulfosuccinate | 1.0 |

| **Phase B** | |
|---|---|
| Water | q.s. ad 100 |
| Sodium Dihydrogenphosphate | 0.5 |
| Disodium Hydrogenphsophate | 0.1 |

The procedure is according to Example 1. Perfume XI is a translucent emulsion.

### Example 12 Perfume XII

| **Phase A** | |
|---|---|
| Water | 7.0 |
| Polyglyceryl-4 Laurate | 8.0 |
| Cyclohexasiloxane & Cyclopentasiloxane | 6.5 |
| Isopropyl Myristate | 1.0 |
| Fragrance mixture with perfume oils (11) and aromatic alcohols (3) (Benzyl Alcohol and 2-Phenoxyethanol) | 14.0 |
| Glycerine | 4.5 |
| Disodium Lauryl Sulfosuccinate | 1.0 |

| **Phase B** | |
|---|---|
| Water | q.s. ad 100 |
| Sodium Dihydrogenphosphate | 0.6 |
| Disodium Hydrogenphsophate | 0.2 |

The procedure is according to Example 1. Perfume XII is transparent.

### Example 13 Perfume XIII

| **Phase A** | |
|---|---|
| Water | 4.0 |
| Polyglyceryl-4 Laurate | 4.5 |
| Isohexadecane | 3.8 |
| Fragrance mixture with perfume oils (6) and aromatic alcohols mixed with Helional *(1.5) | 7.5 |
| Glycerine | 3.0 |
| Disodium Lauryl Sulfosuccinate | 0.4 |

| **Phase B** | |
|---|---|
| Water | q.s. ad 100 |
| Sodium Dihydrogenphosphate | 0.8 |
| Disodium Hydrogenphsophate | 0.2 |

| | |
|---|---|
| * Helional ™ (IFF Inc.)= alpha-methyl-1,3-benzodioxole-5-propanal | |

The procedure is according to Example 1. Perfume XIII is milky white.

### Example 14: Perfume XIV

| **Phase A** | |
|---|---|
| Water | 5.4 |
| Polyglyceryl-4 Laurate | 6.0 |
| Paraffin Oil ISO W 05 | 4.95 |
| Isopropyl Myristate | 2.9 |
| Fragrance mixture with perfume oils (5.0) (CHANTOUNG 668-157941) and aromatic alcohols (1.98) (Benzyl alcohol and 2-Phenoxyethanol) | 6.98 |
| Glycerine | 3.3 |
| Disodium Lauryl Sulfosuccinate | 0.6 |
| PEG-40 Hydrogenated Castor Oil | 1.0 |

| **Phase B** | |
|---|---|
| Water | q.s. ad 100 |
| Sodium Dihydrogenphosphate | 0.71 |
| Disodium Hydrogenphosphate | 0.19 |

The preparation procedure is according to Example 1. Perf transparent emulsion.

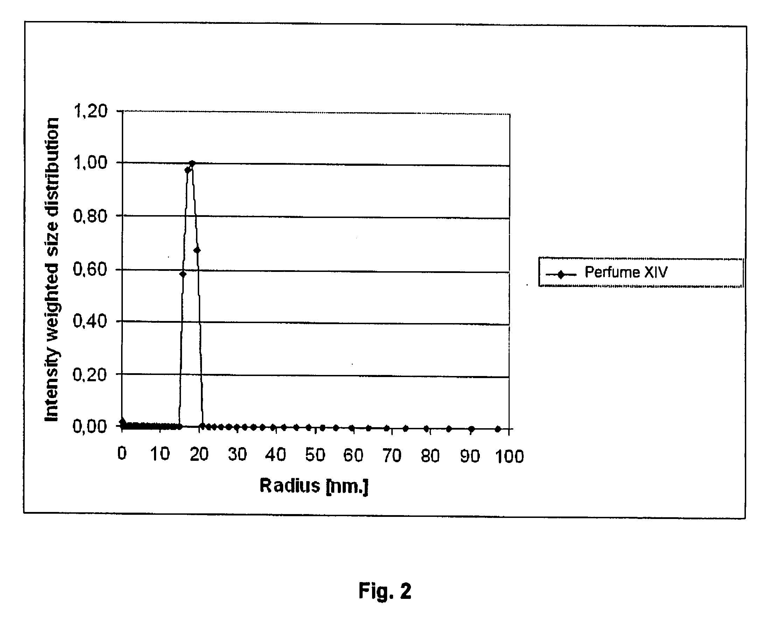

Fig. 2 shows the particle size distribution of the Perfume 14 measured by dynamic light scattering. A very small and narrow signal peak is measured. The mean particle size is smaller than 20 nm.

### Example 15:

### Long-term stability investigations

To evaluate the technical stability of the perfume compositions of the present invention the freeze/thaw stability of emulsions of Example 4 and Example 14 is tested. The freeze/thaw cycle is 24h at -20 °C followed by 24h at +25 °C. 10 cycles in 3 weeks were performed.
Both emulsions remain translucent (Example 4)/transparent (Example14) and no phase separation ("creamy separation") is observed.

Stability of both emulsions is also tested in a SUNTEST simulator (e.g. available from Solar Simulators and Light Sources Data Sheets Atlas Material Testing Technology LLC) using the 24h test and by exposure to natural sun light.
Both emulsions remain translucent (Example 4)/transparent (Example14) and no phase separation ("creamy separation") is observed.

Further investigations with the emulsion of Example 4 show that it becomes white, if the buffer or the first non-ionic emulsifier (e.g.polyglyceryl partial ester) or the anionic emulsifier or the aromatic alcohols are left (a corresponding amount of water was added in each case to give 100 wt%).

If the second non-ionic emulsifier according to the invention (preferably a polyethylene glycol hydrogenated castor oil) is added to the emulsion of Example 4 it becomes transparent. The emulsion of Example 4 is also transparent but not stable over time, if the second non-ionic emulsifier is added, but the buffer or the aromatic alcohols are deleted.

## Claims

1. Perfume composition with reduced alcohol content, comprising a transparent or translucent emulsion with an oil phase and a water phase wherein
(a) the oil phase comprises 25 to 95 wt-% of a perfume oil or perfume oil mixture, and 5 to 75 wt-% of a solvent, the percentages relating to the total weight of the oil phase, wherein the solvent has a log P value of >5 and is selected from Isododecane, Isohexadecane, Isoeicosane, isoparaffine fluids, C₁₃-C₃₀ Alkanes, Hydrogenated Didecene, Hydrogenated Didodecene, Hydrogenated Polydecene, Hydrogenated Polydodecene, Hydrogenated Tridodecene, Hydrogenated Polyisobutene, mineral oils, and a mixture of two or more thereof or the solvent is a linear silicone, a cyclic silicone or a mixture thereof,
(b) the water phase comprises an emulsifier and a buffer system, wherein the emulsifier is a mixture of a first non-ionic emulsifier, an anionic emulsifier and optionally a second non-ionic emulsifier
(c) the amount of the perfume oil or perfume oil mixture is in the range of 1 to 35 wt-%, related to the total weight of the perfume composition;
(d) the perfume composition comprises aromatic substances having a logP value from 0.5 to 2
(e) the amount of mono valent C₂-C₅ alcohols in the perfume composition is in the range of 0 to 5 wt-% , related to the total weight of the perfume composition; and
(f) the water phase and/or oil phase comprise further cosmetically acceptable ingredients.

2. Perfume composition according to claim 1, wherein the oil phase comprises 35 to 85 wt-%, more preferred 40 to 70 wt-% of the perfume oil or perfume oil mixture and 10 to 60 wt-%, more preferred 20 to 45 wt-% of the solvent, related to the total weight of the oil phase.

3. Perfume composition according to claim 1 or 2, wherein the first non-ionic emulsifier is a polyglyceryl partial ester, a glyceryl partial ester, a sorbitan partial ester, a sorbitol partial ester, a carbohydrate ester, a (alkylpoly)glycoside or a mixture thereof, preferably a polyglyceryl partial ester, wherein the carboxylic acid and alcohol parts of the esters are derived from C₆-C₂₂ carboxylic acids and C₆-C₂₂ alcohols, respectively.

4. Perfume composition according to any one of claims 1 to 3, wherein the anionic emulsifier is a sulfate, a sulfonate, a citrate, a sulfosuccinate, a tartrate, a phosphate esterified with C₆ - C₂₂ alcohols or a mixture thereof, preferably a sulfosuccinate.

5. Perfume composition according to any one of claims 1 to 4, wherein the second non-ionic emulsifier is a polyethylene glycol hydrogenated castor oil with 2 to 200 polyethylene glycol units, preferably PEG-40 Hydrogenated Castor Oil, PEG-60 Hydrogenated Castor Oil, Polysorbate 20, Polysorbate 40, Polysorbate 60, Oleth-5, Oleth-10, Oleth-20, PPG-1-PPG-9 Lauryl Glycol Ether or a mixture thereof, preferably PEG-40 Hydrogenated Castor Oil, PEG-60 Hydrogenated Castor Oil or mixtures thereof.

6. Perfume composition according to any one of claims 1 to 5, wherein the range of the total emulsifier is from 0.6 to 20 wt-%, preferably from 1 to 12 wt-% and more preferred from 3 to 10 wt-%, related to the total weight of the perfume composition.

7. Perfume composition according to any one of claims 1 to 6, wherein the solvents in the oil phase are Isohexadecane, C₁₅-C₁₉ Alkanes, C₁₃-C₁₆ Isoparaffins, mineral oils, Isoeicosane, Isododecane or mixtures thereof.

8. Perfume composition according to any one of claims 1 to 7 wherein the aromatic substances have a log P value from 0.5 to 2, preferably from 0.5 to 1.5, more preferred from 0.7 to 1.3.

9. Perfume composition according to claim 8, wherein the aromatic substances are aromatic alcohols, preferably benzyl alcohol, phenoxyethanol, phenoxy propanol, phenoxy propanediol, phenyl ethyl alcohol, methylbenzyl alcohol, anisyl alcohol or mixtures thereof, more preferred benzyl alcohol and/or phenoxy ethanol.

10. Perfume composition according to any one of claims 1 to 9, wherein the buffer system comprises at least one inorganic and one organic component.

11. Perfume composition according to any one of claims 1 to 10, wherein the buffer system is selected from Sodium dihydrogenphosphate/Disodium hydrogenphosphate, Citric acid/Trisodium citrate, Disodium citrate/HCl, Potassium hydrogen phthalate/HCl, (Potassium hydrogen phthalate/HCl)/NaOH, (Disodium citrate/HCl)/NaOH, Potassium dihydrogen phosphate/Disodium hydrogen phosphate, Sodium barbital/HCl, Borax solution/HCl, ((Glycine+NaCl)/HCl)/NaOH, Citric acid/Disodium hydrogen phosphate, Sodium acetate/Acetic acid, Imidazole/HCl, Triethanol - amine + Titriplex III/HCl, Tris(hydroxymethyl)aminomethane/HCl, Sodium carbonate/Sodium hydrogen carbonate or a mixture thereof.

12. Perfume composition according to any one of claims 1 to 11, wherein the buffer system is present in a range of 0.01 to 6 wt-%, related to the total weight of the perfume composition, preferably 0.4 to 3 wt-%, more preferred 0.4 to 1.5 wt-%.

13. Perfume composition according to any one of claims 1 to 12, wherein the amount of mono valent C₂-C₅ alcohols is in the range of 0 to 2.0 wt-% , related to the total weight of the perfume composition, preferably 0 wt-%.

14. Perfume composition according to any one of claims 1 to 13, wherein the oil phase further comprises linear silicones of the Dimethicone type, cyclic silicones, Isononyl Isononanoate, Isopropyl Myristate, Ethylhexyl Palmitate or a mixture thereof.

15. Perfume composition according to any one of claims 1 to 14 wherein the composition is a transparent or translucent composition with a particle diameter of 2 to 35 nm of transparent emulsions and a particle diameter of 35 to 95 nm of translucent emulsions.

## Patentansprüche

1. Parfüm-Zusammensetzung mit reduziertem Alkoholgehalt, umfassend eine transparente oder transluzente Emulsion mit einer Ölphase und einer Wasserphase, wobei
(a) die Ölphase 25 bis 95 Gew.-% eines Parfümöls oder Parfümölgemischs und 5 bis 75 Gew.-% eines Lösungsmittels umfasst, wobei sich die Prozentangaben auf das Gesamtgewicht der Ölphase beziehen, wobei das Lösungsmittel einen logP-Wert von > 5 aufweist und ausgewählt ist aus Isododecane, Isohexadecane, Isoeicosane, flüssigen Isoparaffinen, C₁₃-C₃₀ Alkanes, Hydrogenated Didecene, Hydrogenated Didodecene, Hydrogenated Polydecene, Hydrogenated Polydodecene, Hydrogenated Tridodecene, Hydrogenated Polyisobutene, Mineralölen und einer Mischung aus zwei oder mehr derselben oder das Lösungsmittel ein lineares Silicon, ein cyclisches Silicon oder eine Mischung derselben ist;
(b) die wässrige Phase einen Emulgator und ein Puffersystem umfasst, wobei der Emulgator eine Mischung aus einem ersten nichtionischen Emulgator, einem anionischen Emulgator und gegebenenfalls einem zweiten nichtionischen Emulgator ist;
(c) die Menge des Parfümöls oder Parfümölgemischs, bezogen auf das Gesamtgewicht der Parfüm-Zusammensetzung, im Bereich von 1 bis 35 Gew.-% liegt;
(d) die Parfüm-Zusammensetzung aromatische Substanzen mit einem logP-Wert von 0,5 bis 2 umfasst;
(e) die Menge der einwertigen C₂-C₅-Alkohole in der Parfüm-Zusammensetzung, bezogen auf das Gesamtgewicht der Parfüm-Zusammensetzung, im Bereich von 0 bis 5 Gew.-% liegt; und
(f) die Wasser- und/oder Ölphase weitere kosmetisch annehmbare Inhaltsstoffe umfasst.

2. Parfüm-Zusammensetzung nach Anspruch 1, wobei die Ölphase das Parfümöl oder Parfümölgemisch zu 35 bis 85 Gew.-%, vorzugsweise 40 bis 70 Gew.-% und das Lösungsmittel zu 10 bis 60 Gew.-%, vorzugsweise 20 bis 45 Gew.-% bezogen auf das Gesamtgewicht der Ölphase enthält.

3. Parfüm-Zusammensetzung nach Anspruch 1 oder 2, wobei der erste nichtionische Emulgator ein Polyglycerylpartialester, ein Glycerylpartialester, ein Sorbitanpartialester, ein Sorbitpartialester, ein Kohlenhydratester, ein Alkylpolyglycosid oder eine Mischung derselben, vorzugsweise ein Polyglycerylpartialester ist, wobei Carbonsäureteil und Alkoholteil der Ester von C₆-C₂₂-Carbonsäuren bzw. C₆-C₂₂-Alkoholen abgeleitet sind.

4. Parfüm-Zusammensetzung nach einem der Ansprüche 1 bis 3, wobei der anionische Emulgator ein mit C₆-C₂₂-Alkoholen oder einer Mischung derselben verestertes Sulfat, Sulfonat, Citrat, Sulfosuccinat, Tartrat, Phosphat, vorzugsweise Sulfosuccinat ist.

5. Parfüm-Zusammensetzung nach einem der Ansprüche 1 bis 4, wobei der zweite nichtionische Emulgator ein Polyethylenglycol/hydriertes Rizinusöl mit 2 bis 200 Polyethylenglycol-Einheiten ist, vorzugsweise PEG-40 Hydrogenated Castor Oil, PEG-60 Hydrogenated Castor Oil, Polysorbate 20, Polysorbate 40, Polysorbate 60, Oleth-5, Oleth-10, Oleth-20, PPG-1-PPG-9 Lauryl Glycol Ether oder eine Mischung derselben, vorzugsweise PEG-40 Hydrogenated Castor Oil, PEG-60 Hydrogenated Castor Oil oder Mischungen derselben.

6. Parfüm-Zusammensetzung nach einem der Ansprüche 1 bis 5, wobei der Bereich des gesamten Emulgators, bezogen auf das Gesamtgewicht der Parfüm-Zusammensetzung, 0,6 bis 20 Gew.-%, vorzugsweise 1 bis 12 Gew.-% und mehr bevorzugt 3 bis 10 Gew.-% beträgt.

7. Parfüm-Zusammensetzung nach einem der Ansprüche 1 bis 6, wobei die Lösungsmittel in der Ölphase Isohexadecane, C₁₅-C₁₉ Alkanes, C₁₃-C₁₆ Isoparaffins, Mineralöle, Isoeicosane, Isododecane oder Mischungen derselben sind.

8. Parfüm-Zusammensetzung nach einem der Ansprüche 1 bis 7, wobei die aromatischen Substanzen einen logP-Wert von 0,5 bis 2, vorzugsweise 0,5 bis 1,5 und mehr bevorzugt 0,7 bis 1,3 aufweisen.

9. Parfüm-Zusammensetzung nach Anspruch 8, wobei die aromatischen Substanzen aromatische Alkohole sind, vorzugsweise Benzylalkohol, Phenoxyethanol, Phenoxypropanol, Phenoxypropandiol, Phenylethylalkohol, Methylbenzylalkohol, Anisalkohol oder Mischungen derselben, vorzugsweise Benzylalkohol und/oder Phenoxyethanol.

10. Parfüm-Zusammensetzung nach einem der Ansprüche 1 bis 9, wobei das Puffersystem wenigstens eine anorganische und eine organische Komponente umfasst.

11. Parfüm-Zusammensetzung nach einem der Ansprüche 1 bis 10, wobei das Puffersystem ausgewählt ist aus Sodium dihydrogen phosphate/Disodium hydrogen phosphate, Citric acid/Trisodium citrate, Disodium citrate/HCl, Potassium hydrogen phthalate/HCl, (Potassium hydrogen phthalate/HCl)/ NaOH, (Disodium citrate/HCl)/NaOH, Potassium dihydrogen phosphate/Disodium hydrogen phosphate, Sodium barbital/HCl, Borax Solution/HCl, (Glycine + NaCl)/HCl)/NaOH, Citric acid/Disodium hydrogen phosphate, Sodium acetate/Acetic acid, Imidazole/HCl, Triethanolamine + Titriplex III/HCl, Tris(hydroxymethyl)aminomethane/HCl, Sodium carbonate/Sodium hydrogen carbonate oder einer Mischung derselben.

12. Parfüm-Zusammensetzung nach einem der Ansprüche 1 bis 11, wobei das Puffersystem, bezogen auf das Gesamtgewicht der Parfüm-Zusammensetzung, in einem Bereich von 0,01 bis 6 Gew.-%, vorzugsweise 0,4 bis 3 Gew.-% und mehr bevorzugt 0,4 bis 1,5 Gew.-% vorliegt.

13. Parfüm-Zusammensetzung nach einem der Ansprüche 1 bis 12, wobei die Menge der einwertigen C₂-C₅-Alkohole, bezogen auf das Gesamtgewicht der Parfüm-Zusammensetzung, im Bereich von 0 bis 2,0 Gew.-% und vorzugsweise 0 Gew.-% ist.

14. Parfüm-Zusammensetzung nach einem der Ansprüche 1 bis 13, wobei die Ölphase des Weiteren Silicone vom Dimethicone-Typ, cyclische Silicone, Isononyl Isononanoate, Isopropyl Myristate, Ethylhexyl Palmitate oder eine Mischung derselben umfasst.

15. Parfüm-Zusammensetzung nach einem der Ansprüche 1 bis 14, wobei die Zusammensetzung eine transparente oder transluzente Zusammensetzung mit einem Teilchendurchmesser von 2 bis 35 nm bei transparenten Emulsionen und einem Teilchendurchmesser von 35 bis 95 nm bei transluzenten Emulsionen ist.

## Revendications

1. Composition de parfum ayant une teneur réduite en alcool, comprenant une émulsion transparente ou translucide comportant une phase huile et une phase eau, dans laquelle
(a) la phase huile comprend 25 à 95 % en poids d'une huile de parfum ou d'un mélange d'huile de parfum, et 5 à 75 % en poids d'un solvant, les pourcentages se rapportant au poids total de la phase huile, dans laquelle le solvant a une valeur de logP > 5 et est sélectionné parmi le groupe comprenant l'isododecane, l'isohexadecane, l'isoeicosane, les isoparaffines liquides, les C₁₃-C₃₀ alkanes, l'hydrogenated didecene, l'hydrogenated didodecene, l'hydrogenated polydecene, l'hydrogenated polydodecene, l'hydrogenated tridodecene, l'hydrogenated polyisobutene, les huiles minérales et un mélange de deux ou plus de ceux-ci ou le solvant est un silicone linéaire, un silicone cyclique ou un mélange de ceux-ci,
(b) la phase eau comprend un émulsifiant et un système tampon, moyennant quoi l'émulsifiant est un mélange d'un premier émulsifiant non ionique, d'un émulsifiant anionique et facultativement d'un second émulsifiant non ionique,
(c) la quantité d'huile de parfum ou de mélange d'huile de parfum est comprise entre 1 et 35 % en poids, rapporté au poids total de la composition de parfum,
(d) la composition de parfum comprend des substances aromatiques ayant une valeur de logP comprise entre 0,5 et 2,
(e) la quantité d'alcools monovalents en C₂-C₅ dans la composition de parfum est comprise entre 0 et 5 % en poids, rapporté au poids total de la composition de parfum, et
(f) la phase eau et/ou la phase huile comprennent en plus des adjuvants cosmétiquement acceptables.

2. Composition de parfum selon la revendication 1, dans laquelle la phase huile comprend 35 à 85 % en poids, de préférence 40 à 70 % en poids d'huile de parfum ou de mélange d'huile de parfum et 10 à 60 % en poids, de préférence 20 à 45 % en poids de solvant, rapporté au poids total de la phase huile.

3. Composition de parfum selon la revendication 1 ou 2, dans laquelle le premier émulsifiant non ionique est un ester partiel de polyglycéryl, un ester partiel de glycéryl, un ester partiel de sorbitane, un ester partiel de sorbitol, un ester de carbohydrate, un alkyl polyglycoside ou un mélange de ceux-ci, de préférence un ester partiel de polyglycéryl, moyennant quoi les parties d'acide carboxylique et d'alcool des esters sont dérivés respectivement d'acides carboxyliques en C₆-C₂₂ et d'alcools en C₆-C₂₂.

4. Composition de parfum selon l'une quelconque des revendications 1 à 3, dans laquelle l'émulsifiant anionique est un sulfate, un sulfonate, un citrate, un sulfosuccinate, un tartrate, un phosphate estérifié avec des alcools en C₆-C₂₂ ou un mélange de ceux-ci, de préférence un sulfosuccinate.

5. Composition de parfum selon l'une quelconque des revendications 1 à 4, dans laquelle le second émulsifiant non ionique est une huile de ricin hydrogénée avec du polyéthylène glycol, comportant 2 à 200 unités de polyéthylène glycol, de préférence du PEG-40 hydrogenated castor oil, du PEG-60 hydrogenated castor oil, du polysorbate 20, du polysorbate 40, du polysorbate 60, de l'oleth-5, de l'oleth-10, de l'oleth-20, du PPG-1-PPG-9 lauryl glycol ether ou un mélange de ceux-ci et de manière plus préférée du PEG-40 hydrogenated castor oil, PEG-60 hydrogenated castor oil ou des mélanges de celles-ci.

6. Composition de parfum selon l'une quelconque des revendications 1 à 5, dans laquelle la teneur totale en émulsifiant est de 0,6 à 20 % en poids, de préférence de 1 à 12 % en poids et de manière encore plus préférée de 3 à 10 % en poids, rapporté au poids total de la composition de parfum.

7. Composition de parfum selon l'une quelconque des revendications 1 à 6, dans laquelle les solvants dans la phase huile sont de l'isohexadecane, des C₁₅-C₁₉, alkanes, des C₁₃-C₁₆ isoparaffins, des huiles minérales, de l'isoeicosane, de l'isododecane ou des mélanges de ceux-ci.

8. Composition de parfum selon l'une quelconque des revendications 1 à 7, dans laquelle les substances aromatiques ont une valeur de logP comprise entre 0,5 et 2, de préférence entre 0,5 et 1,5 et de manière plus préférée entre 0,7 et 1, 3.

9. Composition de parfum selon la revendication 8, dans laquelle les substances aromatiques sont des alcools aromatiques, de préférence de l'alcool benzylique, du phénoxyéthanol, du phénoxypropanol, du phénoxy propanediol, de l'alcool phényléthylique, de l'alcool méthylbenzylique, de l'alcool anisylique ou des mélanges de ceux-ci et de manière encore plus préférée de l'alcool benzylique et/ou du phénoxyéthanol.

10. Composition de parfum selon l'une quelconque des revendications 1 à 9, dans laquelle le système tampon comprend au moins une composante minérale et une composante organique.

11. Composition de parfum selon l'une quelconque des revendications 1 à 10, dans laquelle le système tampon est sélectionné parmi le groupe comprenant le sodium dihydrogen phosphate/disodium hydrogen phosphate, citric acid/trisodium citrate, disodium citrate/HCl, potassium hydrogen phthalate/HCl, (potassium hydrogen phthalate/HCl)/ NaOH, (disodium citrate/HCl)/NaOH, potassium dihydrogen phosphate/disodium hydrogen phosphate, sodium barbital/HCl, borax solution/HCl, (glycine + NaCl)/HCl)/NaOH, citric acid/disodium hydrogen phosphate, sodium acetate/acetic acid, imidazole/HCl, triethanolamine + Titriplex III/HCl, tris(hydroxymethyl)aminomethane/HCl, sodium carbonate/sodium hydrogen carbonate ou un mélange d'entre eux.

12. Composition de parfum selon l'une quelconque des revendications 1 à 11, dans laquelle le système tampon est présent en une quantité comprise entre 0,01 et 6 % en poids, rapporté au poids total de la composition de parfum, de préférence entre 0,4 et 3 % en poids et de manière encore plus préférée de 0,4 à 1,5 % en poids.

13. Composition de parfum selon l'une quelconque des revendications 1 à 12, dans laquelle la quantité d'alcools monovalents en C₂-C₅ est comprise entre 0 et 2 % en poids, rapporté au poids total de la composition de parfum, de préférence de 0 % en poids.

14. Composition de parfum selon l'une quelconque des revendications 1 à 13, dans laquelle la phase huile comprend en plus des silicones linéaires du type diméthicone, des silicones cycliques, de l'isononyl isononanoate, de l'isopropyl myristate, de l'éthylhexyl palmitate ou un mélange de ceux-ci.

15. Composition de parfum selon l'une quelconque des revendications 1 à 14, dans laquelle la composition est une composition transparente ou translucide ayant une taille de particules de 2 à 35 nm pour les émulsions transparentes et une taille de particules de 35 à 95 nm pour les émulsions translucides.
